Europäisches Patentamt

(19) European Patent Office

Office européen des brevets

(11) Publication number: **0 454 291 A1**

(12) **EUROPEAN PATENT APPLICATION**

(21) Application number: **91301468.4**

(22) Date of filing: **25.02.91**

(51) Int. Cl.⁵: **A01G 7/00, C12N 1/20,**
**// (C12N1/20, C12R1:41)**

(30) Priority: **26.02.90 US 485202**

(43) Date of publication of application:
**30.10.91 Bulletin 91/44**

(84) Designated Contracting States:
**ES FR IT NL**

(71) Applicant: **W.R. Grace & Co.-Conn.**
**1114 Avenue of the Americas**
**New York New York 10036 (US)**

(72) Inventor: **Paau, Alan S.**
**5405 Jonquil Court**
**Middleton, WI 53562 (US)**

(74) Representative: **Bentham, Stephen et al**
**J.A. Kemp & Co. 14 South Square Gray's Inn**
**London, WC1R 5LX (GB)**

(54) **Process for producing enhanced rhizobium inoculants.**

(57) A process for producing an enhanced
Rhizobium inoculant capable of outcompeting
indigenous low nitrogen-fixing Rhizobium
strains is disclosed. This process involves cul-
turing a Rhizobium strain in a media with seed
meal or seed meal extract, inoculating legume
seeds with the cultured Rhizobium strain and
germinating the legume seeds.

EP 0 454 291 A1

Jouve, 18, rue Saint-Denis, 75001 PARIS

# PROCESS FOR PRODUCING ENHANCED RHIZOBIUM INOCULANTS

The present invention relates to bacteriological symbiosis with legumes. It is particularly related to increasing the competitiveness of novel, high nitrogen-fixing Rhizobium strains.

Legumes have a symbiotic relationship with Rhizobium bacteria. The bacteria inhabit the nodules of legume roots and convert, or "fix," atmospheric nitrogen into biologically useful nitrogen for synthesis of essential biological compounds, such as proteins. Nitrogen fixation by Rhizobium bacteria is also beneficial because nitrogenous nutrients are left in the soil for non-nitrogen-fixing crops that may be planted later. Peas, beans and alfalfa are examples of agriculturally important legumes that are capable of symbiotic relationships with Rhizobium bacteria.

Each different Rhizobium species is capable of forming symbiotic relationships with only a specific legume species. For example, Rhizobium japonicum only nodulates the roots of soybeans. Common agricultural practice is to inoculate either the soil or the legume seeds themselves with an appropriate Rhizobium culture. This inoculation encourages efficient symbiosis, which leads to enhanced legume crop yield.

Because of the importance of increasing legume crop yield, a great deal of research has gone into improving the nitrogen-fixing ability of Rhizobium strains. Unfortunately, while improved Rhizobium strains may exhibit enhanced nitrogen-fixing capacity in laboratory or greenhouse trials, the improved strains are often outcompeted by indigenous Rhizobium strains which may have lesser nitrogen-fixing abilities. Thus introduced, high nitrogen-fixing Rhizobium strains may be ineffective-in increasing crop yield under field conditions because the indigenous, low nitrogen-fixing strains-have evolved to grow more efficiently in that particular soil or environmental condition and may overwhelm the introduced strain.

One way to make introduced, high nitrogen-fixing strains more competitive is to culture the introduced Rhizobium so as to accelerate the formation of the symbiotic relationship with the host legume plant. Legume root exudates contain chemical stimulators which effect the establishment in vitro of the Rhizobium-legume symbiosis. Currier, W. W. and G. A. Strobel, "Chemotaxis of Rhizobium spp. to plant root exudates," Plant Physiol. 57:820-823 (1979); "Characterization and biological activity of trefoil (Lotus corniculatus) chemotactin," Plant Sci. Lett 21:159-166 (1981); "Role of Root exudates on in vitro establishment of symbiosis between Cicer and Rhizobium." In: Symbiotic Nitrogen Fixation (Ed. B.S. Ghai), USG Publishers and Distributors, Ludiana, India, pp 35-42 (1984). Rhizobium bacteria treated with legume root exudates have altered plant hormone production and nodulation characteristics. Garcia-Rodriquez, T., et al., "Effects of legume root exudates on IAA production of Rhizobium Meliloti," Pol. J. Soil Sci. 14:45-52 (1981); Bhagwat, A.A. and J. Thomas, "Legume-Rhizobium interactions: host induced alterations in capsular poly-saccharides and infectivity of cowpea rhizobia," Arch. Micobiol. 140:260-264 (1984); Dazzo, F.B., et al., "Alteration of the trifolin A-binding capsule of Rhizobium tifplii 0403 by enzymes released from clover roots," Appl. Environ. Micobiol. 44:478-490 (1982). Legume root exudate treatment also alters expression of the protein products of the nodC and nodA genes that are essential for the nodulation of legumes, as illustrated by experiments with alfalfa plants. Egelhoff, T. T. and S. R. Long, "Rhizobium meliloti genes: identification of nodDABC gene products, purification of nodA protein, and expression of nodA in Rhizobium melicoti," J. Bacteriol. 164-591-599 (1985).

Because the amount of exudate produced by roots is minimal, the use of root exudates is impractical and cost prohibitive for commercial agricultural applications. It would be advantageous to find another treatment method. The chemical stimulators found in legume roots also may be present in legume seeds. seaton, P. J. and W. W. Currier, "Chemotaxis of Rhizobium spp. to alfalfa root and seed exudates," Plant Physiol. (Suppl.) 63:113 (1979). It was recently reported that washings of alfalfa seed contained factors that stimulated the expression of nod genes of Rhizobium meliloti.

The present invention is a process for enhancing legume crop yield by: culturing a strain of Rhizobium bacteria in a medium containing legume seed meal or legume seed meal extract, inoculating legume seeds with the cultured strain, and germinating the inoculated legume seeds.

An object of the present invention is to provide a process for producing a high nitrogen-fixing Rhizobium strain that can outcompete indigenous Rhizobium strains under field conditions.

One advantage of the present invention is that the process is convenient and economical.

Other objects, advantages and features of the present invention will become apparent from the following specification.

The present invention is directed toward Rhizobium inoculants introduced to a crop field and is intended to enable such introduced strains to compete more effectively with indigenous Rhizobium strains in nodulating legume roots. It has been found that culturing the Rhizobium strains to be introduced with legume seed meal or seed meal extract gives the introduced strain an effective head start in competition with an indigenous strain. The evidence presented here suggests that components of the seed meal "ready" the inoculant strain so that it is better able to compete for the nodulation niches, and further that such strains are effective to increase yield of the legume.

Soybean seed meal is a readily available commodity which can be obtained from agricultural supply stores. The particular strain of the legume from which the seed meal is made is not believed to be critical. Seed meal extract is the filtrate obtained from filtering a mixture of water and seed meal, preferably mixed at a ratio of one part water to two parts seed meal. The seed meal extract thus presumably contains water soluble components of the seed meal itself. The seed used for the seed meal or seed meal extract is that of a compatible host to the particular Rhizobium strain cultured, i.e. soybean seed meal in the case of Rhizobium japonicum.

Rhizobium strains are preferably cultured in a mixture of vermiculite and a nutrient source, preferably a yeast extract - mannitol (YEM) broth containing per liter 10g mannitol, 1g yeast extract, 0.2 g MgSO$_4$.7H$_2$O, 0.2g NaCl, 0.65g K$_2$PO$_4$.3H$_2$O, 6.7 mg FeCl$_3$.6H$_2$O, and 4.2 $\mu$l of HCl. Exfoliated vermiculite is ground in a Wiley mill to a particle size of 45/80 mesh (U.S. Standard Sieves) and autoclaved in small containers with 1.5 ml of YEM broth per g of dry vermiculite. A bacterial inoculant is added to the autoclaved media at the concentration of 10$^2$ to 10$^7$ bacteria per g of vermiculite. If seed meal is added to the vermiculite-nutrient, it is preferably in a ratio of one part seed meal to four parts vermiculite. If seed meal extract is used, it is preferably mixed in a one to one ratio with 2x YEM broth.

The vermiculite-nutrient-inoculant mixture is incubated, preferably at room temperature for 30 days. After appropriate growth, the culture is mixed with damp soybean seeds at the rate of 6 g (wet weight) of vermiculite culture per pound of seeds until the seeds are coated. The coated seeds are germinated in pots filled with vermiculite.

The competitiveness of a Rhizobium strain cultured in a media containing seed meal or seed meal exudate versus a strain cultured without seed meal or seed meal exudate can be measured by using two Rhizobium strains that have differential sensitivity to ampicillin and determining the relative nodule occupancy of each of the two strains. At an ampicillin concentration of 40 $\mu$g per ml of media, Rhizobium japonicum strain IA2875 is sensitive to ampicillin, while Rhizobium japonicum strain K567 is not.

Examples

Example 1:

Rhizobium japonicum K567 strain was cultured in a mixture of four parts vermiculite and YEM broth and one part ground Centennial soybean seed. Strain K567 is a high nitrogen-fixing mutant strain developed by mutation and selection from a wild-type natively competitive strain. Rhizobium japonicum IA2875, a wild-type natively competitive strain, was cultured only in vermiculite and YEM broth. Centennial soybean seeds were coated with a mixture of the two Rhizobium vermiculite cultures and germinated in pots filled with vermiculite.

After three weeks of greenhouse growth, the percentage of soybean nodule occupancy attributable to K567 relative to IA2875 was measured by the differential antibiotic resistance of K567 and IA2875. IA2875 is sensitive to the antibiotic ampicillin, while K567 is resistant. This percentage was compared to the relative nodule occupancy attributable to K567 in a control sample using plants grown from a group of seeds coated with a mixture of K567 and IA2875, in which both strains were cultured in vermiculite and YEM broth without seed meal. The average increase in K567 nodule occupancy in the seed meal treated sample over that found in the control group was 15.63%. The soybean plants grown from seeds inoculated with K567 exposed to seed meal were visibly larger and more vigorous than control group plants.

Example 2:

Rhizobium japonicum K567 was cultured in a mixture of vermiculite, YEM broth and an aqueous extract of Centennial seed meal. Rhizobium japonicum IA2875 was cultured in vermiculite and YEM broth. Centennial soybean seeds were coated with a mixture of the two vermiculite cultures and germinated in pots filled with vermiculite.

After three weeks of greenhouse growth, the percentage of nodule occupancy attributable to K567 in the plants was compared to the percentage of nodule occupancy attributable to K567 in a control group of plants the seeds of which had been coated with a mixture of R567 and IA2875 vermiculite cultures that had not been exposed to the aqueous extract. The average increase in nodule occupancy attributable to seed meal extract treated K567 over that of the control was 16.80%. The soybean plants grown from seeds inoculated with K567 exposed to seed meal extract were again visibly larger than control group plants.

Example 3:

Example 3 was identical to Example 2 except that another variety of soybean seed was treated and Rhizobium japonicum IA2875 was treated with the aqueous extract, not K567.

Rhizobium japonicum IA2875 was cultured in a mixture of vermiculite, YEM broth and an aqueous extract of Williams 82 soybean seed meal. Rhizobium japonicum K567 was cultured in vermiculite and YEM broth. Williams 82 seeds were coated with mixture of the two cultures.

After three weeks of greenhouse growth, the percentage of nodule occupancy attributable to IA2875 was determined by the differential antibiotic sensitivity of IA2875 and K567. This percentage was compared to the percentage of nodule occupancy attributable to IA2875 in plants grown from a control group of seeds coated with a mixture of K567 and IA2875 vermiculite cultures that had not been exposed to seed meal extract. The average increase in nodule occupancy attributable to seed meal extract treated IA2875 over that in the control group was 13.80%. The soybean plants grown from seeds inoculated with IA2875 exposed to seed meal extract were again visibly larger than control group plants.

The above examples thus demonstrate the efficacy of the pre-treatment of Rhizobium inoculant strains with either legume seed meal or legume seed meal extract. Since the data reveals that the relative competitiveness of the two strains used could be altered simply by the addition of the seed meal extract to the fermentation broth, the use of such an additive in the creation of field inoculants will result in improved yield of the subject legume. It is envisioned that the seed meal, or the seed meal extract, can thus simply be added to the fermentation process, either from the beginning or at a late stage in the fermentation process, so that the inoculant bacteria are prepared to compete upon inoculation into the soil.

## Claims

1. A method for enhancing the crop yield of a legume species, comprising the steps of
   (a) exposing a strain of Rhizobium bacteria to a medium containing legume seed meal or an extract thereof; and
   (b) inoculating the legume with the Rhizobium strain from step (a).

2. A method for enhancing the crop yield of a legume species, which comprises
   (a) culturing a strain of Rhizobium bacteria in a medium containing legume seed meal or an aqueous extract thereof; and
   (b) inoculating the legume species with the Rhizobium strain resulting from the culturing.

3. A method as claimed in claim 2, wherein the legume seed meal aqueous extract is one half of the volume of the nutrient source.

4. A method as claimed in claim 1, 2 or 3 wherein the medium containing the seed meal or extract thereof comprises vermiculite and a nutrient source.

5. A method as claimed in claim 4, wherein the vermiculite to seed meal ratio is 4:1.

6. A method as claimed in any one of the preceding claims, wherein the nutrient source is a yeast extract-mannitol broth.

7. A method as claimed in any one of the preceding claims, wherein the legume is inoculated by coating the Rhizobium strain on legume seeds.

8. A method as claimed in any one of the preceding claims, wherein the legume is inoculated by coating the seed of the legume with a vermiculite culture of said Rhizobium strain.

9. A method as claimed in any one of the preceding claims, wherein the legume is soybean and the Rhizobium is Rhizobium japonicum.

10. A medium for the culture of Rhizobium bacteria comprising
    a carrier;
    a nutrient; and
    a quantity of an extract of legume seed meal sufficient to enhance the ability of the Rhizobium cultured in the medium to compete in the field for nodule occupancy.

11. A medium for the culture of Rhizobium bacteria comprising
    a carrier;
    a nutrient; and
    a quantity of legume seed meal sufficient to enhance the ability of the Rhizobium cultured in the medium to compete in the field for nodule occupancy.

12. An inoculant for enhancing the crop yield of a legume species which comprises Rhizobium bacteria from step (a) in claim 1 or 2.

European Patent Office

# EUROPEAN SEARCH REPORT

Application Number

EP 91 30 1468

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 226 394 (AGRACETUS)<br><br>* claims 1-14; example 5 *<br>* page 3, line 4 * | 1,2,4-8, 10-12 | C12N1/20<br>C12R1/41<br>A01G7/00 |
| X | CHEMICAL ABSTRACTS, vol. 100, no. 15,<br>9 April 1984, Columbus, Ohio, US;<br>abstract no. 117658V,<br>A.SHIKIMAKA: 'EFFECT OF ORGANIC ADDITIVES ON RHIZOBIUM SURVIVAL IN LIGNIN'<br>page 229 ; column 2 ;<br>* abstract *<br>& IZV. AKAD. NAUK. MOLD. SSR, SER. BIOL. KHIM. NAUK<br>vol. 6, 1983, SU | 1,2,6, 9-12 | |
| X | CHEMICAL ABSTRACTS, vol. 81, no. 11,<br>16 September 1974, Columbus, Ohio, US;<br>abstract no. 62099V,<br>S.DULATI: 'EFFECT OF ADDITIONAL SHOTS ON GROWTH OF RHIZOBIUM TRIFOLII IN RELATION TO MASS CULTIVATION OF RHIZOBIA'<br>page 353 ; column 2 ;<br>* abstract *<br>& HINDUSTAN ANTIBIOT. BULL.<br>vol. 16, no. 2-3, 1973,<br>pages 84 - 8; | 1,2, 10-12 | TECHNICAL FIELDS SEARCHED (Int. Cl.5)<br><br>C12N<br>C12R<br>A01G |

The present search report has been drawn up for all claims

| Place of search | Date of completion of the search | Examiner |
|---|---|---|
| THE HAGUE | 07 AUGUST 1991 | COUCKE A.O.M. |

EPO FORM 1503 03.82 (P0401)